# EUROPEAN PATENT SPECIFICATION

(11) **EP 2 611 358 B1**
(45) Date of publication and mention of the grant of the patent: **28.10.2020**
(21) Application number: 11773983.9
(22) Date of filing: 01.09.2011
(51) Int. Cl.: A61B 5/087, A61B 5/091, G16H 50/50

(54) **SYSTEM AND APPARATUS FOR AUTOMATICALLY DIAGNOSING EMPHYSEMA**
SYSTEM UND VORRICHTUNG ZUR AUTOMATISCHEN DIAGNOSE EINES EMPHYSEMS
SYSTÈME ET APPAREIL POUR DIAGNOSTIQUER AUTOMATIQUEMENT UN EMPHYSÈME

(30) Priority: 14.02.2011 GB 201102528; 14.02.2011 GB 201102527; 14.02.2011 GB 201102525; 02.09.2010 GB 201014588
(43) Date of publication of application: 10.07.2013
(73) Proprietor: Katholieke Universiteit Leuven, 3000 Leuven (BE)
(72) Inventor: DECRAMER, Marc, 3212 Pellenberg (BE); JANSSENS, Wim, 3010 Kessel-Lo (BE)
(74) Representative: Pronovem
(86) International application number: PCT/BE2011/000055
(87) International publication number: WO 2012/027804

(56) References cited:
- MOHAMMAD MEHDI ZAHMATKESH ET AL: "Is Measurement of Beta Angle in Flow Volume Loop Useful for Diagnosis of Airways Obstruction?", TANAFFOS, vol. 2, no. 5, 1 January 2003 (2003-01-01) , pages 37-42, XP55013825,
- E. NEIL SCHACHTER ET AL: "Smoking and Cotton Dust Effects in Cotton Textile Workers: An Analysis of the Shape of the Maximum Expiratory Flow Volume Curve", ENVIRONMENTAL HEALTH PERSPECTIVES, vol. 66, 1 January 1986 (1986-01-01), pages 145-148, XP55013846,
- M C KAPP ET AL: "The shape of the maximum expiratory flow volume curve", CHEST, vol. 94, 1 January 1988 (1988-01-01), pages 799-806, XP55013777,

## Description

### Background and Summary

### BACKGROUND OF THE INVENTION

### A. Field of the Invention

The present invention relates generally to diagnosing chronic obstructive pulmonary disease (COPD) and estimating the severity thereof, in particular the occurrence of emphysema. More particularly the present invention relates to a system and method for computerized quantification of airway collapse during forced expiration or total exhalation. Such airway collapse is correlated with the presence of emphysema that has been verified on computer tomography (CT) scan and that is due to the loss of alveolar attachments in emphysema.

Moreover the present invention provides a computerized apparatus for detection of emphysema , a computerized system for detection of emphysema or a method of computer-aided detection of emphysema. This can also be used for quantifying the severity of emphysema in patients with COPD by an automated analysis of the amount (volume) and/or speed (flow) of a total or forced exhalation or the processing of the graphics of a pneumotachograph of a total or forced exhalation. Such measurements are obtainable by a spirometer.

The apparatus and system of present invention correlates airway collapse during forced expiration in COPD with presence and severity of emphysema. It was demonstrated that airway collapse during forced expiration in COPD correlates with presence and severity of emphysema.

Several documents are cited throughout the text of this specification. Each of the documents herein (including any manufacturer's specifications, instructions etc.) are herby incorporated by reference; however, there is no admission that any document cited is indeed prior art of the present invention.

### B. Description of the Related Art

Worldwide, COPD ranked as the sixth leading cause of death in 1990. It is projected to be the fourth leading cause of death worldwide by 2030 due to an increase in smoking rates and demographic changes in many countries. COPD is the 4th leading cause of death in the U.S., and the economic burden of COPD in the U.S. in 2007 was $42.6 billion in health care costs and lost productivity. The diagnosis of COPD requires lung function tests such as respirometry.

Emphysema is a long-term, progressive disease of the lung. It is included in a group of diseases called COPD. Emphysema is called an obstructive lung disease because the destruction of lung tissue around smaller airways, called alveoli, makes these airways unable to hold their functional shape upon exhalation.

Forced expiratory volumes and flows or the expiratory volumes and flows of total exhalation are used to diagnose COPD. However the current methods and systems in the art do not accurately distinguish airway obstruction from emphysema and are thus not suitable for accurate distinguishing emphysema from COPD and for estimating the severity of emphysema. Such current methods are described in following papers: M. M. ZAHMATKESH ET AL: "Is Measurement of Beta Angle in Flow Volume Loop Useful for Diagnosis of Airways Obstruction?",TANAFFOS, vol. 2, no. 5, 1 January 2003, pages 37-42, XP055013825; M. C. KAPP ET AL: "The shape of the maximum expiratory flow volume curve", CHEST, vol. 94, 1 January 1988, pages 799-806, XP055013777; E. N. SCHACHTER ET AL: "Smoking and Cotton Dust Effects in Cotton Textile Workers: An Analysis of the Shape of the Maximum Expiratory Flow Volume Curve", ENVIRONMENTAL HEALTH PERSPECTIVES, vol. 66, 1 January 1986, pages 145-148, XP055013846.

Thus, there is a need in the art for more accurate diagnosis systems and methods to detect emphysema and assess the severity of emphysema in a group of diseases called COPD.

Present invention provides such by computerized quantification of airway collapse during forced expiration and demonstrates correlations with the presence of emphysema on CT scan.

### SUMMARY OF THE INVENTION

The invention is related to a system and to an apparatus as disclosed in the appended claims.

An embodiment of the present invention solves the problems of the related art of inaccurate diagnosing of emphysema by providing an apparatus for diagnosing emphysema , the apparatus comprising **a)** a device for receiving and sensing forced or total expiration of the respiratory system of a patient and further comprising **b)** a calculator or signal processor calculating the flow volume curve or relationship from said forced or total airway expiration, characterized in that the calculator or signal processor comprises **1)** a first calculating means for calculating the flow-volume curve or the flow-volume relationship and **2)** a second calculating means for automatically calculating the angle in the expiratory flow-volume curve between the two best fitting linear regression curves on the flow-volume curve.

In accordance with the purpose of the invention, as embodied and broadly described herein, the invention is broadly drawn to an apparatus for diagnosing emphysema whereby the apparatus having **a)** a signal input to receive electrical signals produced by volume sensor and flow sensors of a device for receiving and sensing forced or total expiration of the respiratory system of a patient and **b)** a calculator or signal processor calculating the flow volume curve or relationship from said forced or total airway expiration, wherein the calculator or signal processor comprises **1)** a first calculating means for calculating the flow-volume curve or the flow-volume relationship and **2)** a second calculating means for automatically calculating the angle in the expiratory flow-volume curve between the two best fitting linear regression curves on the flow-volume curve corresponding to entire exhalation flow rate and exhalation volume of a patients respiratory system.

This apparatus of present invention or described above can further comprise calculating means for calculating the peak-to-surface area. Moreover in the apparatus of present invention or as described above the signal processor or calculator further can comprise a mathematical model to compare said angle with that of a plurality of chronic obstructive pulmonary disease (COPD) patients with no emphysema. Moreover the signal processor or calculator further comprising a mathematical model to compare said angle with that of a plurality of COPD patients affected with emphysema. In yet another embodiment the signal processor or calculator further comprises a mathematical model to compare said angle with that of a plurality of COPD patients affected with a defined seriousness or with defined progress of emphysema. Furthermore the signal processor or calculator in the apparatus here above described can further comprise a mathematical model to compare said angle with that of a control or the signal processor comprises a mathematical model that is described to automatically calculate expiratory airway collapse due to loss of alveolar attachments in emphysema.

The device for receiving and sensing forced or total expiration of the respiratory system of a patient can be for present invention a spirometer.

Another embodiment of present invention is a system for diagnosing an emphysema disorder in a chronic obstructive pulmonary disease (COPD) patient, the system comprising: 1) a sampling device to obtain total expiration or exhalation of the respiratory system of the subject; 2) a detection device generating flow and volume data of said total expiration or exhalation; and 3) a computer loaded with a model to calculate the flow-volume loop; the two best fitting linear regression curves on flow-volume loop from peak flow to end of expiration and the angle between both regression lines and further loaded with a flow-volume loop reference profile for a emphysema disorder; wherein the computer receives and compares subject's flow-volume loop profile with the reference flow-volume loop profile.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description. It is to be understood that both the foregoing general description and the following detailed description are exemplary and explanatory only and are not restrictive of the invention, as claimed.

In an advantageous embodiment, the apparatuses according to the present invention further comprises a device for receiving and sensing forced or total expiration of the respiratory system of a patient is a spirometer.

### Detailed Description

### DETAILED DESCRIPTION OF EMBODIMENTS OF THE INVENTION

The following detailed description of the invention refers to the accompanying drawings. The same reference numbers in different drawings identify the same or similar elements. Also, the following detailed description does not limit the invention. Instead, the scope of the invention is defined by the appended claims and equivalents thereof.

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn to scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it doe not exclude other elements or steps. It is thus to be interpreted as specifying the presence of the stated features, integers, steps or components as referred to, but doe not preclude the presence or addition of one or more other features, integers, steps or components, or groups thereof. Thus, the scope of the expression "a device comprising means A and B" should not be limited to the devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

Forced expiratory volumes and flows are used to diagnose COPD and estimate the severity thereof. They do not accurately distinguish airway obstruction from emphysema. We investigated whether a computerized detection and quantification of airway collapse during forced expiration and measurement of the amount (volume) and/or speed (flow) of a total or forced exhalation correlated with the presence of emphysema on CT scan. The amount (volume) and/or speed (flow) of a total or forced exhalation can be assessed by a spirometer. A spirometer is an apparatus for measuring the volume of air inspired and expired by the lungs. It is a precision differential pressure transducer for the measurements of respiration flow rates. The spirometer records the amount of air and the rate of air that is breathed in and out over a specified period of time. An incentive spirometer is used to help patients improve the functioning of their lungs. Tank-type spirometer works as the same principle as the gasometer. A canister of soda is usually attached to absorb carbon dioxide and a kymograph trace is produced to record changes in total volume gas. From this, vital capacity, tidal volume, breathing rate and ventilation rate (=tidal volume x breathing rate) can be calculated. From the overall decline on the graph, the oxygen uptake can also be measured

### EXAMPLES

Example 1: 513 patients with >15 pack-years and > 50 years were enrolled. Electronic data of the best spirometry (ATS/ERS criteria) were used to calculate the two best fitting linear regression curves on flow-volume loops from peak flow to end of expiration. The angle between both regression lines (AC) was used as surrogate marker of airway collapse. AC was related to diffusing capacity over alveolar ventilation (KCO), another functional variable known to be associated with emphysema but NOT accessible with spirometry in a general practise. AC was also related to semi-quantitative visual scores of emphysema on CT.

In 93% of patients (n=477) the computer model resulted in a correct quantification of mean AC, 156°±7° in healthy subjects (n=138) and 135°±10° in COPD patients (n=339). In subjects with FEV1/FVC ratio > 0.7, ACs were not different between emphysema and non-emphysema subgroups. In COPD patients however, the mean AC in the emphysema subgroup (n=238) was significantly lower as compared to the non-emphysema group (n=101) (130° vs. 145°), even when stratifying for GOLD stage (p<0.0001). Multivariate analysis retained KCO as the best indicator of emphysema on CT scan. When considering only spirometry, AC was clearly the best predictor of visually scored emphysema (R2=0.43, p< 0.0001), whereas other variables such as FEV1, FEV1/FVC ratio, time of expiration, peak flow to surface ratio and SVC-VC difference did not further contribute to the regression model. Finally, receiver operating curves defined 130° as best cut-off for emphysema with a specificity of 95% and sensitivity of 52% (AUC=0.82, p < 0.0001).

Airway collapse on expiratory flow reflected by AC, correlates with severity of emphysema. A cut-off of 130° may identify emphysema in general practice with high certainty. Other embodiments of the invention will be apparent to those skilled in the art from consideration of the specification and practice of the invention disclosed herein. It is intended that the specification and examples be considered as exemplary only. Each and every claim is incorporated into the specification as an embodiment of the present invention. Thus, the claims are part of the description and are a further description and are in addition to the preferred embodiments of the present invention. Each of the claims set out a particular embodiment of the invention. The following terms are provided solely to aid in the understanding of the invention.

### Example 2: a calculation process.

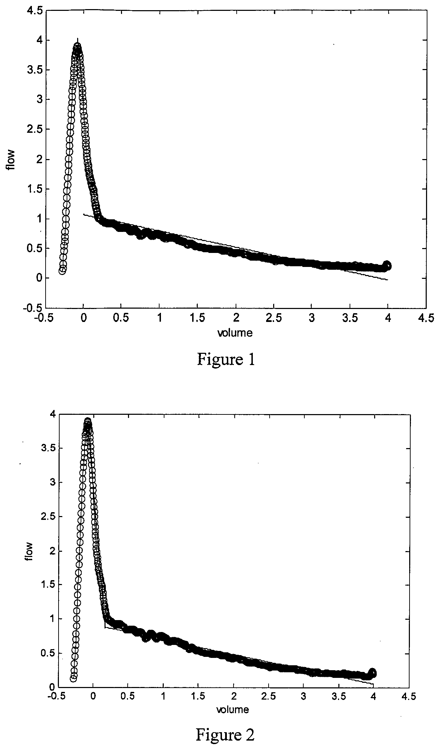

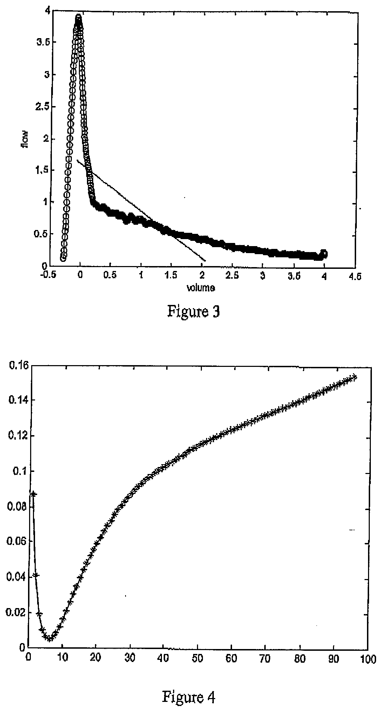

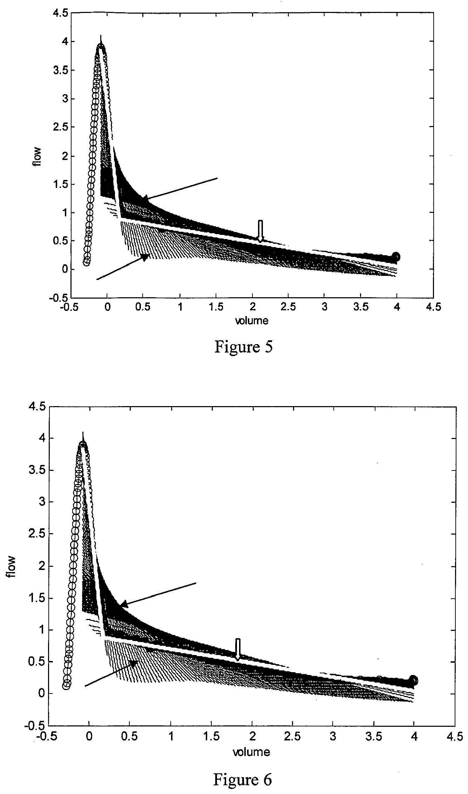

As first COPD indicator the peak-to-surface value of flow-volume curve or the flow-volume relationship is calculated. The peak-to-surface value is the peak value divided by the surface under the measured curve. As second COPD indicator the angle between two lines is calculated, which lines describe the point cloud between the peak and the maximal volume in the meaning that the mean square error between the data and fitted curve are minimal. Since it is initially not known which angle points to select or which portion of the data to fit with curve one and which of the remaining portion to fit with curve 2, the automated program makes different runs. For each run the automated program selects a new angle point with a 10 points interval starting at peakflow. For instance 1) for the first run (run 1) we select the angle point 1x10 (= 10 samples) after the peak and a fit run is carried out 2) for the second run (run 2) an angle point is selected at 2x10 (= 20 samples) of the peak and a fit is carried out and 3) such operations with the same or similar sample gaps are repeated. Such examples are demonstrated in the figures 1 - 3. Figure 1 demonstrates the best fitting linear regression curves at run 2 (the angle point at 2x10 (= 20 samples) of the peak). The fit is acceptable but not yet optimal. Figure 2 demonstrates the fit for the repetitive runs at run 6 (the angle point being selected at 6 x 10 (= 60 samples) of the peak). This already demonstrates a best fit. Figure 3 demonstrates a fit for run 50; i.e. if we select an angle point at 50x10 (= 500 samples) of the peak. Here the fit is suboptimal. Figure 4 demonstrates the goodness of fit of all runs and shows the mean square error for al the runs. Hereby is clearly demonstrated that the best total fit occurred at run 6 (angle point selected at 6x10 (= 60 samples) of the peak. An overview of all runs is provided in figure 5. Figure 5 demonstrates the fits for all runs (run 1 to run 5000) which is designated by the single line arrow →. The fit of the best run is designated by double line arrow This concerns run six with angular point at 6*10 = 60 sample points of the peak. The MSE (mean square error) was hereby minimal. This figure 5 demonstrates the calculated two best fitting linear regression curves on flow-volume loops from peak flow to end of expiration and the angle between both regression lines (AC).

Particular and preferred aspects of the invention are set out in the accompanying independent and dependent claims. Features from the dependent claims may be combined with features of the independent claims and with features of other dependent claims as appropriate and not merely as explicitly set out in the claims. Thus, the claims following the detailed description are hereby expressly incorporated into this detailed description, with each claim standing on its own as a separate embodiment of this invention.

### Drawing Description

### BRIEF DESCRIPTION OF THE DRAWINGS

The present invention will become more fully understood from the detailed description given herein below and the accompanying drawings which are given by way of illustration only, and thus are not limitative of the present invention, and wherein:
Figure 1: concerns a COPD analysis by the measurement of the amount (volume) and/or speed (flow) of air of a forced exhalation and displays the fit through two linear regression line (defining a relationship between the flow and the volume) on 20 samples points of the peak of the flow volume curve from said forced airway expiration.
Figure 2: concerns a COPD analysis by the measurement of the amount (volume) and/or speed (flow) of air of a forced exhalation and displays the fit through two linear regression line (defining a relationship between the flow and the volume) on 60 samples points of the peak of the flow volume curve from said forced airway expiration.
Figure 3: concerns a COPD analysis by the measurement of the amount (volume) and/or speed (flow) of air of a forced exhalation and displays the fit through two linear regression line (defining a relationship between the flow and the volume) on 500 samples points of the peak of the flow volume curve from said forced airway expiration.
Figure 4: displays the MSE (mean square error) of different runs. A minimum is reached at run 6, e.g. if we select an angular point on 6*10 = 60 sampling points from the peak. Figure 5: demonstrates the fits for all runs (designated by the single line arrow →). The fit of the best run is designated by double line arrow. This concerns run six with angular point at 6*10 = 60 sample points of the peak. The MSE (mean square error) was hereby minimal. This figure demonstrates the calculated two best fitting linear regression curves on flow-volume loops from peak flow to end of expiration and the angle between both regression lines (AC)

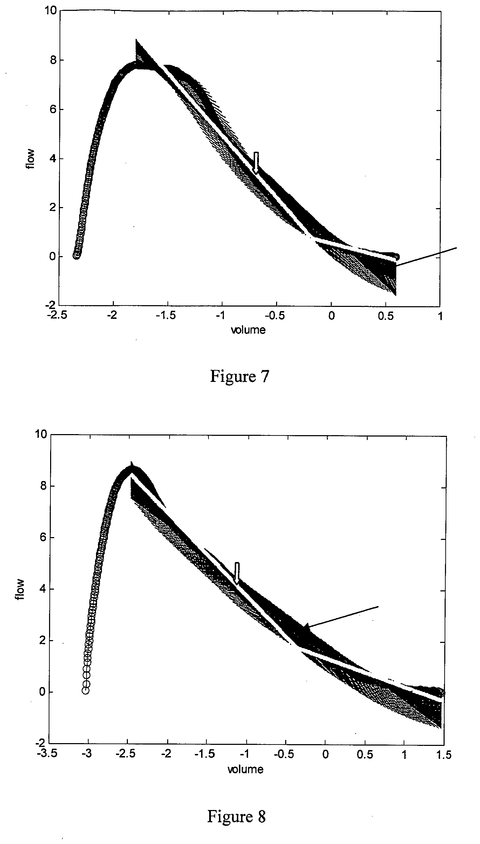

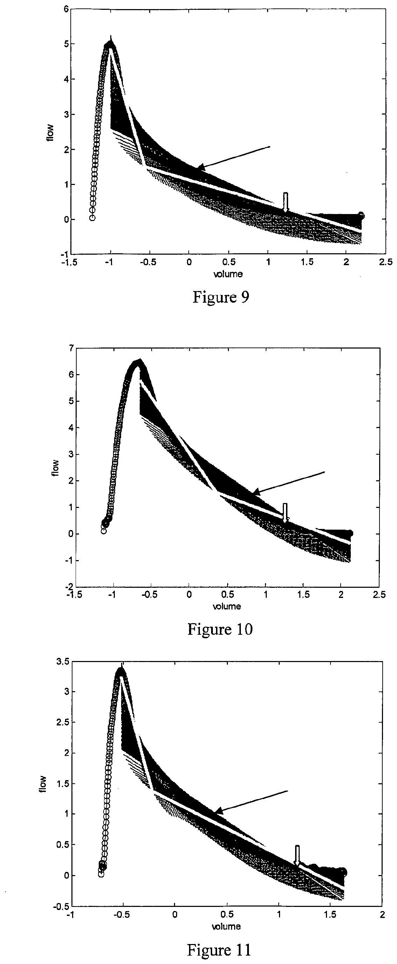

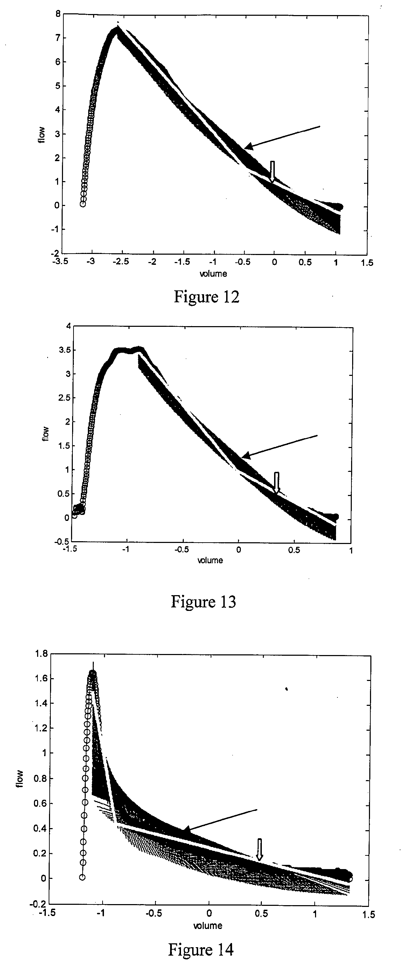

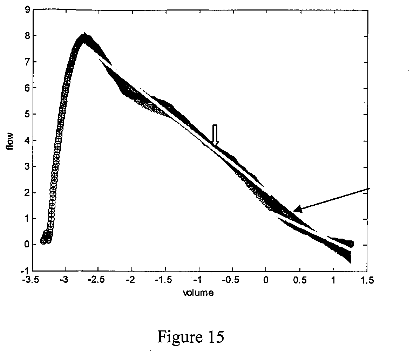

Figures 6 to 15 concern measurement of the amount (volume) and/or speed (flow) (designated by the single line arrow →) of air of a forced exhalation and displays the fit on 10 different patients. These figures demonstrate the calculated two best fitting linear regression curves (designated by double line arrow) on flow-volume loops from peak flow to end of expiration and the angle between both regression lines (AC)

| | | |
|---|---|---|
| • | Figure 6 : | Peak_to_surface = 1.3893 and Angle = 109.4244 |
| • | Figure 7 : | Peak_to_surface = 0.7073 and Angle = 154.0281 |
| • | Figure 8 : | Peak_to_surface: 0.5547 and Angle: 154.5251 |
| • | Figure 9 : | Peak_to_surface: 1.3299 and Angle: 132.5744 |
| • | Figure 10 : | Peak_to_surface: 0.9722 and Angle: 149.8245 |
| • | Figure 11 : | Peak_to_surface: 1.5545 and Angle: 140.2217 |
| • | Figure 12 : | Peak_to_surface: 0.5386 and Angle: 159.2410 |
| • | Figure 13 : | Peak_to_surface: 0.9283 and Angle: 158.3582 |
| • | Figure 14 : | Peak_to_surface: 2.1694 and Angle: 117.7351 |
| • | Figure 15 : | Peak_to_surface: 0.4691 and Angle: 166.5198 |

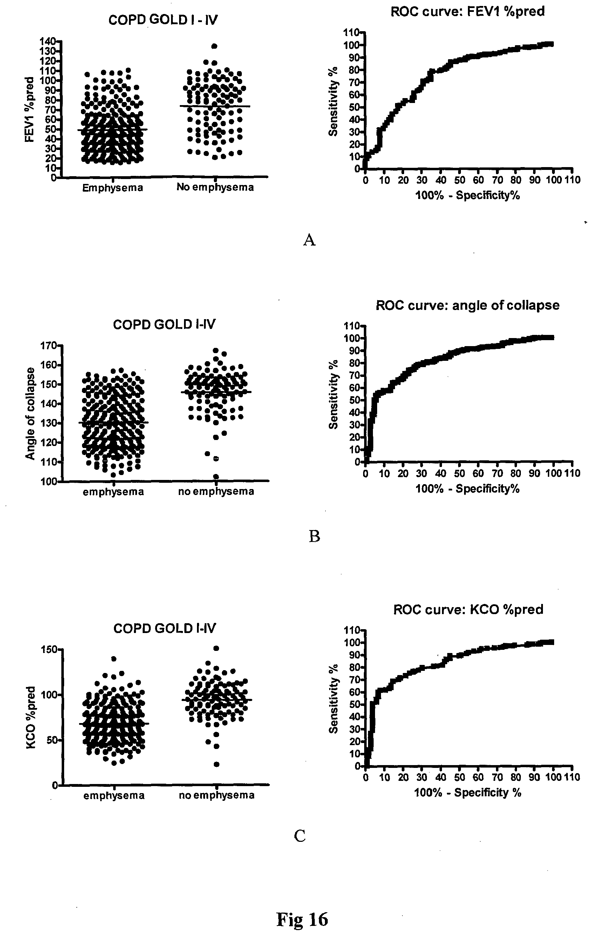

Figure 16 a - c provides an overview of COPD analysis on the patient as described in example 1. The receiver operating characteristic demonstrated that 130° provides the best cut off value to diagnose emphysema in the flow-volume curve that represents the measurement of the amount (volume) and/or speed (flow) of air of a forced exhalation of a COPD patient (figure 16a). The specificity was 95%. The sensitivity was 52% (area under the curve AUC= 0.82, p< 0.0001) There are none or very few patients with an angle smaller than 130° who do not have the emphysema disorder as could be confirmed via a visual score system on CT -images. Figure 16b demonstrates that lower FEV1% relates to more patients with emphysema but is also common in COPD patients without emphysema and can therefore not be used for discrimination. Figure 16c demonstrates that diffusing capacity corrected for alveolar volume, a previously accepted non spirometry-derived predictor for emphysema, is almost equally efficient as the angle of collaps, a variable which can be obtained from every spirometry.

Accordingly, the present invention provides a system for diagnosing a emphysema disorder in a chronic obstructive pulmonary disease (COPD) patient, the system comprising: 1) a sampling device to obtain total expiration or exhalation of the respiratory system of the subject; 2) a detection device generating flow and volume data of said total expiration or exhalation; and 3) a computer loaded a model to calculate the flow-volume loop; the two best fitting linear regression curves on flow-volume loop from peak flow to end of expiration and the angle between both regression lines and further loaded with a flow-volume loop reference profile for a emphysema disorder; wherein the computer receives and compares subject's flow-volume loop profile with the reference flow-volume loop profile. The present invention also provides a system for diagnosing a emphysema disorder in a chronic obstructive pulmonary disease (COPD) patient, the system comprising: a sampling device to obtain total expiration or exhalation of the respiratory system of the subject; a detection device generating flow and volume data of said total expiration or exhalation; and a computer loaded model to calculate the flow-volume loop; the two best fitting linear regression curves on flow-volume loop from peak flow to end of expiration and the angle between both regression lines and further loaded with a flow-volume loop reference profile for an emphysema disorder; wherein the computer receives and compares subject's flow-volume loop profile with the reference flow-volume loop profile.

This invention accordingly provides the advantage that emphysema disorder can be distinguished in a chronic obstructive pulmonary disease (COPD) patient group.

In an advantageous embodiment, the system according to the present invention and described here above comprise a calculating means for calculating the peak-to-surface area. In any of the different embodiments it comprises a mathematical model that compares said angle with that of plurality of chronic obstructive pulmonary disease (COPD) patients with no emphysema. Hereby the mathematical model can compare the angle with that of plurality of COPD patients affected with emphysema; the mathematical model can compare said angle with that of plurality of COPD patients affected with a defined seriousness or with defined progress of emphysema; the mathematical model compares said angle with that of a control and/or the mathematical model compares automatically calculates expiratory airway collapse due to loss of alveolar attachments in emphysema.

An embodiment of the present invention also provides an apparatus for diagnosing emphysema , the apparatus comprising a) a device for receiving and sensing forced or total expiration of the respiratory system of a patient and further comprising b) a calculator or signal processor calculating the flow volume curve or relationship from said forced or total airway expiration, characterized in that the calculator or signal processor comprises 1) a first calculating means for calculating the flow-volume curve or the flow-volume relationship and 2) a second calculating means for automatically calculating the angle in the expiratory flow-volume curve between the two best fitting linear regression curves on flow-volume curve, whereby the signal processor or calculator further comprising a mathematical model to compare said angle with that of a COPD disorder patient subgroups or with a reference angle of one or more such COPD disorder subgroups.

The present invention further provides an apparatus for diagnosing emphysema whereby the apparatus having a) a signal input to receive electrical signals produced by volume sensor and flow sensors of a device for receiving and sensing forced or total expiration of the respiratory system of a patient and b) a calculator or signal processor calculating the flow volume curve or relationship from said forced or total airway expiration, wherein the calculator or signal processor comprises 1) a first calculating means for calculating the flow-volume curve or the flow-volume relationship and 2) a second calculating means for automatically calculating the angle in the expiratory flow-volume curve between the two best fitting linear regression curves on the flow-volume curve corresponding to entire exhalation flow rate and exhalation volume of a patients respiratory system, whereby the signal processor or calculator further comprises a mathematical model to compare said angle with that of a COPD disorder patient subgroups or with a reference angle of one or more such COPD disorder subgroups. These apparatuses can comprise a signal processor or calculator comprising a mathematical model to compare said angle with that of plurality of chronic obstructive pulmonary disease (COPD) patients with no emphysema or with a reference angle of COPD but no emphysema.

In an embodiment of the apparatus the signal processor or calculator comprises a mathematical model to compare said angle with that of plurality of COPD patients affected with emphysema or with a reference angle for COPD and emphysema

In another embodiment of the apparatus the signal processor or calculator comprises a mathematical model to compare said angle with that of plurality of COPD patients affected with a defined seriousness or with defined progress of emphysema.

In another embodiment of the apparatus the signal processor or calculator comprises a mathematical model to compare said angle with that of a control.

Furthermore the apparatuses according to the present invention can be characterized in that the apparatus comprises a signal processor comprising a mathematical model that is described to automatically calculate expiratory airway collapse due to loss of alveolar attachments in emphysema.

It is an object of the present invention to provide such apparatus with a calculating means that is adapted for calculating the peak-to-surface area. This provides the surprising advantage that emphysema disorder can be distinguished in a chronic obstructive pulmonary disease (COPD) patient group by a device for receiving and sensing forced or total expiration of the respiratory system of a patient for instance by a spirometer. A simple, fast and accurate technology to discover emphysema disorder and advocate a proper corresponding treatment.

the seriousness or progress of emphysema; to define a respiratory therapy medication; to define emphysema or for diagnosing emphysema without radiological imaging such as CT scanning; to define emphysema or for diagnosing emphysema whereby an angle smaller than 140° is indicative for emphysema; to define emphysema or for diagnosing emphysema whereby an angle smaller than 135° is indicative for emphysema; to define emphysema or for diagnosing emphysema whereby an angle smaller than 130° is indicative for emphysema; to define emphysema or for diagnosing emphysema whereby an angle smaller than 125° is indicative for emphysema and to define emphysema or for diagnosing emphysema whereby an angle smaller than 120° is indicative for emphysema.

In an embodiment of the invention is also provided an apparatus for diagnosing emphysema , the apparatus comprising a) a device for receiving and sensing forced or total expiration of the respiratory system of a patient and further comprising b) a calculator or signal processor calculating the flow volume curve or relationship from said forced or total airway expiration, characterized in that the calculator or signal processor comprises 1) a first calculating means for calculating the flow-volume curve or the flow-volume relationship and 2) a second calculating means for automatically calculating the angle in the expiratory flow-volume curve between the two best fitting linear regression curves on flow-volume curve, , whereby the signal processor or calculator further comprising a mathematical model to compare said angle with that of a COPD disorder patient subgroups or with a reference angle of one or more such COPD disorder subgroups.

The present invention further provides an apparatus for diagnosing emphysema whereby the apparatus having a) a signal input to receive electrical signals of an electrical signals produced by volume sensor and flow sensors of a device for receiving and sensing forced or total expiration of the respiratory system of a patient • and b) a calculator or signal processor calculating the flow volume curve or relationship from said forced or total airway expiration, characterized in that the calculator or signal processor comprises 1) a first calculating means for calculating the flow-volume curve or the flow-volume relationship and 2) a second calculating means for automatically calculating the angle in the expiratory flow-volume curve between the two best fitting linear regression curves on the flow-volume curve corresponding to entire exhalation flow rate and exhalation volume of a patients respiratory system, whereby the signal processor or calculator further comprising a mathematical model to compare said angle with that of a COPD disorder patient subgroups or with a reference angle of one or more such COPD disorder subgroups. These apparatuses can comprise a signal processor or calculator comprising a with mathematical model to compare said angle with that of plurality of chronic obstructive pulmonary disease (COPD) patients with no emphysema or with a reference angle of COPD but no emphysema.

In an embodiment of the apparatus the signal processor or calculator comprises a mathematical model to compare said angle with that of plurality of COPD patients affected with emphysema or with a reference angle for COPD and emphysema
In another embodiment of the apparatus the signal processor or calculator comprises a mathematical model to compare said angle with that of plurality of COPD patients affected with a defined seriousness or with defined progress of emphysema.

In another embodiment of the apparatus the signal processor or calculator comprises a mathematical model to compare said angle with that of a control.

Furthermore the apparatuses according to the present invention can be characterized in that the apparatus comprises a signal processor comprising a mathematical model that is described to automatically calculate expiratory airway collapse due to loss of alveolar attachments in emphysema.

It is an object of the present invention to provide such apparatus with a calculating means that is adapted for calculating the peak-to-surface area. This provides the surprising advantage that emphysema disorder can be distinguished in a in a chronic obstructive pulmonary disease (COPD) patient group by a device for receiving and sensing forced or total expiration of the respiratory system of a patient for instance by a spirometer. A simple, fast and accurate technology to discover emphysema disorder and advocate a proper corresponding treatment.

The present invention also provides uses of the an apparatuses according to present invention as described in this application here above to define emphysema or for diagnosing emphysema without radiological imaging such as CT scanning; to define emphysema or for diagnosing emphysema without radiological imaging such as CT scanning; to define the seriousness or progress of emphysema; to define a respiratory therapy medication; to define emphysema or for diagnosing emphysema whereby an angle smaller than 140° is indicative for emphysema; to define emphysema or for diagnosing emphysema whereby an angle smaller than 135° is indicative for emphysema; to any one of the claims 1 to 8, to define emphysema or for diagnosing emphysema whereby an angle smaller than 130° is indicative for emphysema; to define emphysema or for diagnosing emphysema whereby an angle smaller than 125° is indicative for emphysema and/or to define emphysema or for diagnosing emphysema whereby an angle smaller than 120° is indicative for emphysema.

## Claims

1. A system for diagnosing an emphysema disorder in a chronic obstructive pulmonary disease (COPD) patient, the system comprising:
1) a sampling device to obtain total expiration or exhalation of the respiratory system of the patient;
2) a detection device generating flow and volume data of said total expiration or exhalation; and
3) a computer loaded with
- a model to calculate :
• the flow-volume curve;
• the two best fitting linear regression lines on the flow-volume curve from peak flow to end of expiration, and
• the angle between both regression lines
- and further loaded with a flow-volume curve reference profile for an emphysema disorder;
wherein the computer receives and compares the patient's flow-volume curve profile with the reference flow-volume curve profile.

2. The system according to claim 1, wherein said model is configured to determine said two best fitting regression lines by
• Calculating a first linear regression line on a first portion of said flow volume curve, said first portion extending between the peak flow point of said curve and a chosen point of said curve,
• Calculating a second linear regression line on a second portion of said flow volume curve, said second portion extending between said chosen point and the end-of-expiration point of the curve,
• Calculating said first and second lines for a plurality of chosen points,
• Determining said best fitting lines as the lines which produce the best total fit on said two portions of the flow volume curve.

3. The system according to claim 1 or 2, further comprising calculating means for calculating the peak-to-surface area.

4. The system according to any of the previous claims 1 to 3, whereby a mathematical model compares said angle with that of plurality of chronic obstructive pulmonary disease (COPD) patients with no emphysema, or with that of plurality of COPD patients affected with emphysema, or with that of plurality of COPD patients affected with a defined seriousness or with defined progress of emphysema.

5. The system according to any of the previous claims 1 to 3, whereby a mathematical model compares said angle with that of a control.

6. The system according to any of the previous claims 1 to 3, whereby a mathematical model automatically calculates expiratory airway collapse due to loss of alveolar attachments in emphysema.

7. The system according to any of the previous claims 1 to 3, whereby an angle smaller than 130° is indicative for emphysema.

8. An apparatus for diagnosing emphysema whereby the apparatus having
a) a signal input to receive electrical signals produced by volume sensors and flow sensors of a device for receiving and sensing forced or total expiration of the respiratory system of a patient and
b) a calculator or signal processor configured to calculate the expiratory flow volume curve from said forced or total airway expiration, wherein the calculator or signal processor comprises :
1) a first calculating means for calculating the flow-volume curve and
2) a second calculating means for automatically calculating the angle in the flow-volume curve between the two best fitting linear regression curves on the flow-volume curve corresponding to entire exhalation flow rate and exhalation volume of a patients respiratory system,
whereby the signal processor or calculator further comprises a mathematical model to compare said angle with a reference angle of one or more COPD disorder patient subgroups.

9. The apparatus according to claim 8, wherein said apparatus for diagnosing emphysema comprises said device for receiving and sensing forced or total expiration of the respiratory system of a patient.

10. The apparatus according to claim 8 or 9, wherein said model is configured to determine said two best fitting regression lines by
• Calculating a first linear regression line on a first portion of said flow volume curve, said first portion extending between the peak flow point of said curve and a chosen point of said curve,
• Calculating a second linear regression line on a second portion of said flow volume curve, said second portion extending between said chosen point and the end-of-expiration point of the curve,
• Calculating said first and second lines for a plurality of chosen points,
• Determining said best fitting lines as the lines which produce the best total fit on said two portions of the flow volume curve.

11. The apparatus according to any one of the previous claims 8 to 10, wherein said one more subgroups are chosen from the group consisting of :
• a plurality of chronic obstructive pulmonary disease (COPD) patients with no emphysema,
• a plurality of COPD patients affected with emphysema
• a plurality of COPD patients affected with a defined seriousness or with defined progress of emphysema.

12. The apparatus according to any one of the previous claims 8 to 9, whereby the signal processor or calculator further comprising a mathematical model to compare said angle with that of a control.

13. The apparatus according to any one of the previous claims 8 to 9, **characterized in that** the apparatus comprises a signal processor comprising a mathematical model that is described to automatically calculate expiratory airway collapse due to loss of alveolar attachments in emphysema.

14. The apparatus according to any one of the previous claims 8 to 9, further comprising calculating means for calculating the peak-to-surface area.

15. The apparatus according to any one of the previous claims 8 to 9, whereby the device for receiving and sensing forced or total expiration of the respiratory system of a patient is a spirometer.

## Patentansprüche

1. Ein System zur Diagnose einer Emphysemstörung bei einem Patienten mit chronischer obstruktiver Lungenerkrankung (COPD), wobei das System Folgendes umfasst:
1) ein Probenahmegerät, um eine vollständige Ausatmung oder Exhalation des Atmungssystems des Patienten zu erzielen;
2) ein Detektionsgerät, das Fluss- und Volumendaten der vollständigen Ausatmung oder Exhalation generiert; und
3) einen Computer, geladen mit
- einem Modell, um Folgendes zu berechnen:
• eine Fluss-Volumen-Kurve;
• die zwei am besten passenden Regressiongeraden auf der Fluss-Volumen-Kurve vom Spitzenfluss zum Ende der Exspiration, und
• den Winkel zwischen beiden Regressionsgeraden,
- und weiter geladen mit einem Referenzprofil einer Fluss-Volumen-Kurve für eine Emphysemstörung;
wobei der Computer das Profil der Fluss-Volumen-Kurve des Patienten empfängt und mit dem Referenzprofil der Fluss-Volumen-Kurve vergleicht.

2. Das System nach Anspruch 1, wobei das besagtes Modell konfiguriert ist, um die zwei besagten am besten passenden Regressionsgeraden zu bestimmen durch:
• Berechnen einer ersten linearen Regressionsgeraden aus einem ersten Abschnitt der besagten Fluss-Volumen-Kurve, wobei sich der besagte erste Abschnitt zwischen dem Spitzenflusspunkt der besagten Kurve und einem ausgewählten Punkt der Kurve erstreckt,
• Berechnen einer zweiten linearen Regressionsgeraden aus einem zweiten Abschnitt der besagten Fluss-Volumen-Kurve, wobei sich der besagte zweite Abschnitt zwischen dem besagten ausgewählten Punkt und dem Punkt des Endes der Exspiration auf der Kurve erstreckt,
• Berechnen der besagten ersten und zweiten Geraden aus einer Vielzahl von ausgewählten Punkten,
• Bestimmen der besagten am besten passenden Geraden als die Geraden, die die beste gesamte Passung auf den besagten zwei Abschnitten der Fluss-Volumen-Kurve erzeugen.

3. Das System nach Anspruch 1 oder 2, weiter umfassend Berechnungsmittel zur Berechnung des Spitzen-zu-Oberflächen-Bereichs.

4. Das System nach einem der vorhergehenden Ansprüche 1 bis 3, wobei ein mathematisches Modell den besagten Winkel mit demjenigen der Vielzahl von Patienten mit chronischer obstruktiver Lungenerkrankung (COPD) ohne Emphysemoder mit demjenigen der Vielzahl von COPD-Patienten, die ein Emphysem aufweisen, vergleicht, oder mit demjenigen der Vielzahl von COPD-Patienten, die von einer definierten Schwere oder von einem definierten Fortschreiten eines Emphysems betroffen sind.

5. Das System nach einem der vorhergehenden Ansprüche 1 bis 3, wobei ein mathematisches Modell den besagten Winkel mit demjenigen einer Kontrolle vergleicht.

6. Das System nach einem der vorhergehenden Ansprüche 1 bis 3, wobei ein mathematisches Modell automatisch das Kollabieren des Exspirationsluftwegs aufgrund des Verlusts von alveolaren Ansätzen im Emphysem berechnet.

7. Das System nach einem der vorhergehenden Ansprüche 1 bis 3, wobei ein Winkel von weniger als 130° ein Emphysem anzeigt.

8. Eine Vorrichtung zur Diagnose eines Emphysems, wobei die Vorrichtung Folgendes umfasst:
a) eine Signaleingabe, um elektrische Signale zu empfangen, die von Volumensensoren und Flusssensoren eines Geräts zum Empfang und Messen von erzwungener oder vollständiger Exspiration des Atmungssystems eines Patienten produziert ist,
b) einen Rechner oder einen Signalprozessor, der konfiguriert ist um die exspiratorische Fluss-Volumen-Kurve aus der besagten erzwungenen oder gesamten Luftwegexspiration zu berechnen,
wobei der Rechner oder Signalprozessor Folgendes umfasst:
1) ein erstes Berechnungsmittel, um die Fluss-Volumen-Kurve zu berechnen, und
2) ein zweites Berechnungsmittel, um automatisch den Winkel in der Fluss-Volumen-Kurve zwischen den zwei am besten passenden Kurven der Regressionskurven auf der Fluss-Volumen-Kurve zu berechnen, der der gesamten Exhalationsflussrate und dem Exhalationsvolumen eines Atmungssystems von Patienten entspricht,
wobei der Signalprozessor oder Rechner weiter ein mathematisches Modell umfasst, um den besagten Winkel mit einem Referenzwinkel eines oder mehrerer Patientenuntergruppen mit COPD-Störung zu vergleichen.

9. Die Vorrichtung nach Anspruch 8, wobei die besagte Vorrichtung zur Diagnose eines Emphysems das besagtes Gerät zum Empfang und Messen von erzwungener oder vollständiger Exspiration des Atmungssystems eines Patienten umfasst.

10. Die Vorrichtung nach Anspruch 8 oder 9, wobei das besagtes Modell konfiguriert ist, um die zwei am besten passenden Regressionsgeraden zu bestimmen durch:
• Berechnen einer ersten linearen Regressionsgeraden aus einem ersten Abschnitt der besagten Fluss-Volumen-Kurve, wobei sich der besagte Abschnitt zwischen dem Spitzenflusspunkt der besagten Kurve und einem ausgewählten Punkt der besagten Kurve erstreckt,
• Berechnen einer zweiten linearen Regressionsgeraden aus einem zweiten Abschnitt der besagten Fluss-Volumen-Kurve, wobei sich der besagte zweite Abschnitt zwischen dem besagten ausgewählten Punkt und dem Punkt des Endes der Exspiration der Kurve erstreckt,
• Berechnen der besagten ersten und zweiten Geraden aus einer Vielzahl von ausgewählten Punkten,
• Bestimmen der besagten am besten passenden Geraden als die Geraden, die die beste gesamte Passung auf den besagten zwei Abschnitten der Fluss-Volumen-Kurve erzeugen.

11. Die Vorrichtung nach einem der vorhergehenden Ansprüche 8 bis 10, wobei die besagte eine oder die besagten mehreren Untergruppen ausgewählt sind aus der Gruppe, bestehend aus:
• einer Vielzahl von Patienten mit chronischer obstruktiver Lungenerkrankung (COPD) ohne Emphysem,
• einer Vielzahl von COPD-Patienten, die ein Emphysem aufweisen,
• eine Vielzahl von COPD-Patienten, die von einer definierten Schwere oder von einem definierten Fortschreiten eines Emphysems betroffen sind.

12. Die Vorrichtung nach einem der vorhergehenden Ansprüche 8 bis 9, wobei der Signalprozessor oder Rechner weiter ein mathematisches Modell umfasst, um den besagten Winkel mit demjenigen einer Kontrolle zu vergleichen.

13. Die Vorrichtung nach einem der vorhergehenden Ansprüche 8 bis 9, **dadurch gekennzeichnet dass** die Vorrichtung einen Signalprozessor umfasst, umfassend ein mathematisches Modell, das beschrieben ist, um automatisch ein Kollabieren des exspiratorischen Luftwegs, aufgrund des Verlusts von alveolaren Ansätzen im Emphysem zu berechnen.

14. Die Vorrichtung nach einem der vorhergehenden Ansprüche 8 bis 9, weiter umfassend Berechnungsmittel zur Berechnung des Spitzen-zu-Oberflächen-Bereichs.

15. Die Vorrichtung nach einem der vorhergehenden Ansprüche 8 bis 9, wobei das Gerät zum Empfang und Messen von erzwungener oder vollständiger Exspiration des Atmungssystems eines Patienten ein Spirometer ist.

## Revendications

1. Système pour diagnostiquer un trouble d'emphysème chez un patient atteint de bronchopneumopathie chronique obstructive (BPCO), le système comprenant :
1) un dispositif d'échantillonnage pour obtenir l'expiration ou l'exhalation totale du système respiratoire du patient ;
2) un dispositif de détection générant des données de débit et de volume de ladite expiration ou exhalation totale ; et
3) un ordinateur chargé avec
- un modèle pour calculer :
• la courbe débit-volume ;
• les deux droites de régression linéaire d'ajustement sur la courbe débit-volume du débit de crête à la fin de l'expiration, et
• l'angle entre les deux droites de régression
- et en outre chargé avec un profil de référence de courbe débit-volume pour un trouble d'emphysème ;
dans lequel l'ordinateur reçoit et compare le profil de courbe débit-volume du patient au profil de courbe débit-volume de référence.

2. Système selon la revendication 1, dans lequel ledit modèle est configuré pour déterminer lesdites deux droites de régression d'ajustement par
• calcul d'une première droite de régression linéaire sur une première partie de ladite courbe débit-volume, ladite première partie s'étendant entre le point de débit de crête de ladite courbe et un point choisi de ladite courbe,
• calcul d'une deuxième droite de régression linéaire sur une deuxième partie de ladite courbe débit-volume, ladite deuxième partie s'étendant entre ledit point choisi et le point de fin d'expiration de la courbe,
• calcul desdites première et deuxième droites pour une pluralité de points choisis,
• détermination desdites droites d'ajustement comme étant les droites qui produisent le meilleur ajustement total sur lesdites deux parties de la courbe débit-volume.

3. Système selon la revendication 1 ou 2, comprenant en outre un moyen de calcul pour calculer l'aire de pic.

4. Système selon l'une quelconque des revendications précédentes 1 à 3, dans lequel un modèle mathématique compare ledit angle à celui d'une pluralité de patients atteints de bronchopneumopathie chronique obstructive (BPCO) sans emphysème, ou à celui de d'une de patients atteints de BPCO affectés d'un emphysème, ou à celui d'une pluralité de patients atteints de BPCO affectés avec une gravité définie ou avec une progression définie d'emphysème.

5. Système selon l'une quelconque des revendications précédentes 1 à 3, dans lequel un modèle mathématique compare ledit angle à celui d'un témoin.

6. Système selon l'une quelconque des revendications précédentes 1 à 3, dans lequel un modèle mathématique calcule automatiquement un collapsus expiratoire des voies respiratoires dû à la perte de fixations alvéolaires dans l'emphysème.

7. Système selon l'une quelconque des revendications précédentes 1 à 3, dans lequel un angle inférieur à 130° est indicatif d'un emphysème.

8. Appareil pour diagnostiquer un emphysème, l'appareil comportant
a) une entrée de signal pour recevoir des signaux électriques produits par des capteurs de volume et des capteurs de débit d'un dispositif pour recevoir et détecter l'expiration forcée ou totale du système respiratoire d'un patient et
b) un calculateur ou un processeur de signal configuré pour calculer la courbe débit-volume expiratoire à partir de ladite expiration forcée ou totale des voies respiratoires,
dans lequel
le calculateur ou processeur de signal comprend :
1) un premier moyen de calcul pour calculer la courbe débit-volume et
2) un deuxième moyen de calcul pour calculer automatiquement l'angle dans la courbe débit-volume entre les deux courbes de régression linéaire d'ajustement sur la courbe débit-volume correspondant aux débit d'exhalation et volume d'exhalation entiers du système respiratoire d'un patient,
dans lequel le processeur de signal ou le calculateur comprend en outre un modèle mathématique pour comparer ledit angle à un angle de référence d'un ou plusieurs sous-groupes de patients atteints de trouble BPCO.

9. Appareil selon la revendication 8, dans lequel ledit appareil pour diagnostiquer un emphysème comprend ledit dispositif pour recevoir et détecter l'expiration forcée ou totale du système respiratoire d'un patient.

10. Appareil selon la revendication 8 ou 9, dans lequel ledit modèle est configuré pour déterminer lesdites deux droites de régression d'ajustement par
• calcul d'une première droite de régression linéaire sur une première partie de ladite courbe débit-volume, ladite première partie s'étendant entre le point de débit de crête de ladite courbe et un point choisi de ladite courbe,
• calcul d'une deuxième droite de régression linéaire sur une deuxième partie de ladite courbe débit-volume, ladite deuxième partie s'étendant entre ledit point choisi et le point de fin d'expiration de la courbe,
• calcul desdites première et deuxième droites pour une pluralité de points choisis,
• détermination desdites droites d'ajustement comme étant les droites qui produisent le meilleur ajustement total sur lesdites deux parties de la courbe débit-volume.

11. Appareil selon l'une quelconque des revendications précédentes 8 à 10, dans lequel lesdits un ou plusieurs sous-groupes sont choisis parmi le groupe consistant en:
• une pluralité de patients atteints de bronchopneumopathie chronique obstructive (BPCO) sans emphysème,
• une pluralité de patients atteints de BPCO affectés d'un emphysème
• une pluralité de patients atteints de BPCO affectés avec une gravité définie ou avec une progression définie d'emphysème.

12. Appareil selon l'une quelconque des revendications précédentes 8 à 9, dans lequel le processeur de signal ou le calculateur comprend en outre un modèle mathématique pour comparer ledit angle à celui d'un témoin.

13. Appareil selon l'une quelconque des revendications précédentes 8 à 9, **caractérisé en ce que** l'appareil comprend un processeur de signal comprenant un modèle mathématique qui est décrit pour calculer automatiquement un collapsus expiratoire des voies respiratoires dû à la perte de fixations alvéolaires dans l'emphysème.

14. Appareil selon l'une quelconque des revendications précédentes 8 à 9, comprenant en outre un moyen de calcul pour calculer l'aire de pic.

15. Appareil selon l'une quelconque des revendications précédentes 8 à 9, dans lequel le dispositif pour recevoir et détecter l'expiration forcée ou totale du système respiratoire d'un patient est un spiromètre.
